# EUROPÄISCHE PATENTSCHRIFT

(11) **EP 0 584 397 B1**
(45) Veröffentlichungstag und Bekanntmachung des Hinweises auf die Patenterteilung: **17.06.1998**
(21) Anmeldenummer: 92114797.1
(22) Anmeldetag: 28.08.1992
(51) Int. Cl.: A61N 1/39

(54) **Defibrillator**
Defibrillator
Défibrillateur

(43) Veröffentlichungstag der Anmeldung: 02.03.1994
(73) Patentinhaber: Pacesetter AB, 175 84 Järfälla (SE)
(72) Erfinder: Högnelid, Kurt, S-137 38 Västerhaninge (SE); Nilsson, Kenth, S-184 60 Akersberga (SE)
(74) Vertreter: Lettström, Richard Wilhelm

(56) Entgegenhaltungen:
- EP-A- 0 383 732
- DE-A- 3 020 479
- FR-A- 1 090 545
- FR-A- 2 156 856
- FR-A- 2 437 219

## Beschreibung

Die Erfindung betrifft einen Defibrillator mit einer Ladekapazität, die an ihren beiden Seiten zum Aufladen an eine Ladeschaltung schaltbar ist und zum Defibrillieren eines Herzens über eine steuerbare Schalteinrichtung mit mindestens zwei im Bereich des Herzens angeordneten Elektroden verbindbar ist.

Ein derartiger, aus der US-A-4 800 883 bekannter Defibrillator ist zur Implantation in dem Körper eines Patienten vorgesehen. Der bekannte Defibrillator enthält eine Ladekapazität bestehend aus zwei Kondensatoren, die zur Aufladung auf eine vorgegebene Spannung an einer Ladeschaltung angeschlossen sind. Die beiden Kondensatoren sind ferner über eine Schalteinrichtung bestehend aus vier, in einer Brückenschaltung angeordneten Schaltern mit zwei am Herzen des Patienten plazierten Elektroden verbunden. Zur Defibrillation des Herzens wird die Ladekapazität zunächst auf eine vorgegebene Spannung aufgeladen und anschließend über die Schalteinrichtung mit den Elektroden am Herzen verbunden, so daß sich die Ladekapazität mit einem Entladestrom über das Herzgewebe entlädt. Indem die vier Schalter der Schalteinrichtung abwechselnd paarweise geöffnet und geschlossen werden, wird der Entladestrom durch das Herzgewebe in eine Mehrzahl von aufeinanderfolgenden Teilströmen mit alternierender Stromrichtung aufgeteilt.

Der die Defibrillierung zustande bringende Strom durch das Herzgewebe ist von der Ladespannung der Ladekapazität und dem elekrischen Widerstand des Herzgewebes zwischen den Elektroden abhängig. Zu Beginn der Entladung der Ladekapazität weist der Strom seinen höchsten Wert auf, um dann exponentiell abzuklingen. Um eine effektive Defibrillation des Herzens zu erhalten, muß der Strom durch das Herzgewebe während einer bestimmten Dauer einen bestimmten Mindestwert überschreiten. Aus diesem Grund wird die Ladespannung für die Ladekapazität so gewählt, dass der Strom zu Beginn des Ladevorgangs ausreichend gross ist, um erst nach Ablauf der bestimmten Dauer unter den Mindestwert abzusinken. Der den Mindestwert übersteigende Anteil des Stromes ist im Hinblick auf eine effektive Defibrillation nicht nur ohne Nutzen, sondern kann darüberhinaus auch aufgrund des hohen Anfangswerts zu Schädigungen des Herzgewebes führen.

Aus der DE-A-30 20 479 ist ein Relais bekannt, bei dem eine bestimmte Menge einer leitenden Flüssigkeit wie z.B. Quecksilber zwischen zwei Kontakten angeordnet ist und unter der Einwirkung eines elektromechanischen Wandlers in Form eines piezoelektrischen Antriebselements steht, das durch elektrische Ansteuerung aus einer Ruhestellung in eine Arbeitsstellung auslenkbar ist. In der Arbeitsstellung des piezoelektrischen Antriebselements wird die Flüssigkeit derart gegen die Wirkung ihrer Oberflächenspannung deformiert, dass sie eine Verbindung der Kontakte herstellt.

Aus der FR-A-2 437 219 ist ein Defibrillator bekannt, bei dem eine Schalteinrichtung zur Steuerung zweier Schalter, jeder zwischen zwei unbeweglichen Kontakten, vorgesehen ist.

Der Erfindung liegt die Aufgabe zugrunde, bei einem Defibrillator die Entstehung von allzu hohen Enladeströmen zu verhindern, ohne dass jedoch dadurch die Effektivität der Defibrillierung beeinflusst wird.

Gemäss der Erfindung wird die Aufgabe dadurch gelöst, dass bei dem Defibrillator der eingangs angegebenen Art die steuerbare Schalteinrichtung zwei voneinander elektrisch isolierte Kontakte aufweist, zwischen denen ein gegen die Wirkung einer inneren Rückstellkraft deformierbares und unter der Einwirkung eines elektromechanischen Wandlers stehendes Schaltstück in der Weise angeordnet ist, dass das Schaltstück bei einer vorgegebenen Auslenkung des elektromechanischen Wandlers die beiden Kontakte miteinander leitend verbindet und bei einer von der vorgegebenen Auslenkung ausgehenden Änderung der Auslenkung aufgrund der davon abhängigen Formänderung des Schaltstücks den elektrischen Übergangswiderstand zwischen den Kontakten, durch Änderung der in Kontakt stehenden Fläche der Kontakte verändert, und dass an dem elektromechanischen Wandler ein Steuerimpulsgenerator angeschlossen ist, der Einschaltimipulse mit einer zum Einstellen des gewünschten elektrischen Widerstands zwischen den Kontakten veränderbaren Impulshöhe erzeugt.

Im Unterschied zu dem aus der DE-OS 30 20 479 bekannten Relais lässt sich mit der steuerbaren Schaltereinrichtung des erfindungsgemässen Defibrillators der Strom durch die Schaltereinrichtung nicht nur ein- und ausschalten, sondern darüberhinaus auch durch Einstellen des gewünschten elektrischen Widerstands zwischen den Kontakten der steuerbaren Schalteinrichtung bezüglich der Stromhöhe steuern.

Entsprechend einer vorteilhaften Weiterbildung des erfindungsgemässen Defibrillators ist vorgesehen, dass die Impulshöhe des Einschaltimpulses während der Impulsdauer in der Weise variiert, dass der elektrische Widerstand zwischen den beiden Kontakten während der Impulsdauer abnimmt. Dadurch wird erreicht, dass der Entladestrom der Ladekapazität und damit der Strom durch das Herzgewebe zu Beginn des Entladevorgangs, wenn die Ladespannung der Ladekapazität am höchsten ist, auf einen für das Herzgewebe unschädlichen Wert begrenzt wird; in dem Masse, wie die Ladespannung der Ladekapazität durch die Entladung abnimmt, kann der elektrische Widerstand zwischen den beiden Kontakten verringert werden.

Die Änderung des elektrischen Widerstands zwischen den beiden Kontakten kann im einfachsten Fall auf den von der Auslenkung des elektromechanischen Wandlers abhängigen Formänderungen des Kontaktstücks beruhen. Eine weitaus bessere Steuerung des elektrischen Widerstands wird in vorteilhafter Weise dadurch erreicht, daß das Schaltstück bei unterschiedlichen Auslenkstellungen des elektromagnetischen Wandlers unterschiedlich große Flächen oder unterschiedliche Bereiche der Kontakte bedeckt. Dabei erfolgt die Änderung des elektrischen Widerstands hauptsächlich durch die Änderung des Übergangswiderstands zwischen dem Kontaktstück und den Kontakten.

In diesem Zusammenhang bestehen die Kontakte vorzugsweise aus einem Widerstandsmaterial, wodurch der Grad der Widerstandsänderung in Abhängigkeit von der Auslenkung des elektromechanischen Wandlers verstärkt wird.

Das Schaltstück besteht vorzugsweise aus einer vorgegebenen Menge einer leitenden Flüssigkeit. Beispiele für solche Flüssigkeiten sind Quecksilber, Gallium, Galliumlegierungen, wie z.B. Ga-In und Ga-In-Sn oder ein Elektrolyt. Für den Fall, daß der erfindungsgemäße Defibrillator als implantierbares Gerät ausgebildet ist, ergibt sich dabei der Vorteil, daß aufgrund der Körperwärme der Schmelzpunkt für die Flüssigkeit vergleichsweise hoch gewählt werden kann, so daß an Stelle von Quecksilber weniger giftige oder ungiftige Substanzen verwendet werden können.

Der elektromechanische Wandler besteht vorzugsweise aus einem piezoelektrischen, elektromagnetischen, magnetostriktiven oder elektrostriktiven Antriebselement.

Zur Erläuterung der Erfindung wird im folgenden auf die Figuren der Zeichnung Bezug genommen. Im einzelnen zeigen
- FIG 1: ein vereinfachtes Blockschaltbild des erfindungsgemäßen Defibrillators mit einer steuerbaren Schalteinrichtung,
- FIG 2: ein Ausführungsbeispiel für die steuerbare Schalteinrichtung mit einem elektromechanischen Wandler in Ruhestellung,
- FIG 3: das Ausführungsbeispiel nach Figur 2, wobei sich der elektromechanische Wandler in einer Auslenkstellung befindet,
- FIG 4: eine alternative Ausbildung der steuerbaren Schalteinrichtung nach den Figuren 2 und 3 und
- FIG 5: in einem Diagramm den Ausgangsstrom des Defibrillators mit und ohne Strombegrenzung durch die steuerbare Schalteinrichtung.

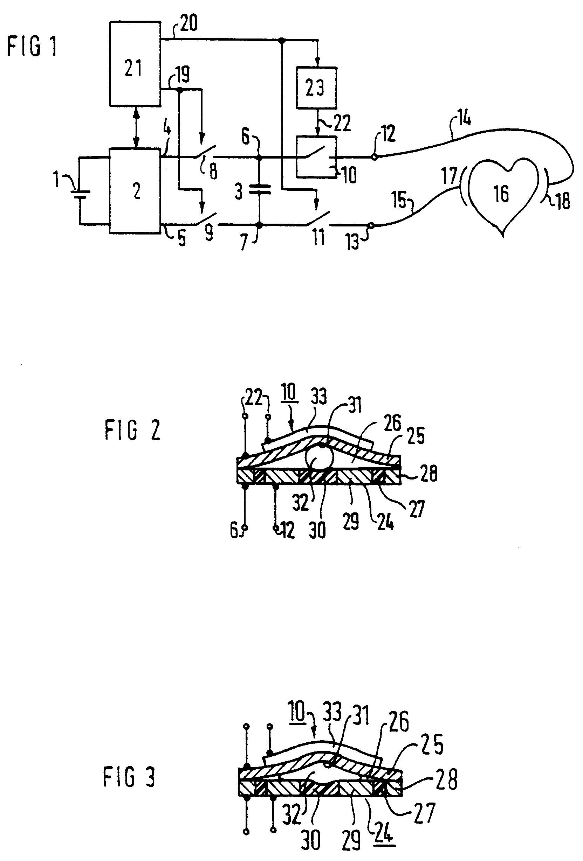

Der in Figur 1 als Blockschaltbild dargestellte Defibrillator enthält eine Spannungsquelle 1 in Form einer Batterie, die an einer Ladeschaltung 2 für eine Ladekapazität 3 angeschlossen ist. Die Ladeschaltung 2 erzeugt an ihren Ausgangsanschlüsen 4 und 5 eine vorgegebene Ladespannung, auf die die Ladekapazität 3 aufgeladen wird, wenn sie an ihren beiden Seiten 6 und 7 über zwei steuerbare Schalter 8 und 9 mit den Ausgangsanschlüssen 4 und 5 der Ladeschaltung 2 verbunden wird. Die beiden Seiten 6 und 7 der Ladekapazität 3 sind ferner über zwei weitere Schalter 10 und 11 einer steuerbaren Schalteinrichtung mit jeweils zwei Elektrodenanschlüssen 12 und 13 verbindbar, an denen über jeweils eine Elektrodenleitung 14 bzw. 15 zwei im Bereich des Herzens 16 eines Patienten angeordnete Elektroden 17 und 18 angeschlossen sind. Die steuerbaren Schalter 8,9 und 11 sind vorzugsweise als Halbleiterschalter ausgebildet und über Steuerausgänge 19 und 20 einer Steuerschaltung 21 ansteuerbar. Der mit 10 bezeichnete Schalter, dessen Aufbau untenstehend an Hand der Figuren 2,3 und 4 näher erläutert wird, ist an seinem Steuereingang 22 über einen Steuerimpulsgenerator 23 mit der Steuerschaltung 21 verbunden.

Der in Figur 1 gezeigte steuerbare Schalter 10 weist ein erstes plattenförmiges Gehäuseteil 24 und ein zweites schalenförmiges Gehäuseteil 25 auf, die beide einen Hohlraum 26 einschließen. Das plattenförmige Gehäuseteil 24 besteht aus einer Isolierplatte 27, die an ihrem äußeren Rand mit einem äußeren Kontaktring 28 versehen ist und einen dazu konzentrisch angeordneten inneren Kontaktring 29 enthält. Das aus Metall bestehende schalenförmige Gehäuseteil 25 ist im Bereich des äußeren Kontaktrings 28 ringförmig auf diesem aufgeschweist. Das schalenförmige Gehäuseteil 25 mit dem äußeren Kontaktring 28 und der innerer Kontaktring 29 bilden die Kontakte des Schalters 10 und sind bei dem Defibrillator nach Figur 1 mit der Seite 6 der Ladekanazität 3 bzw. dem Elektrodenanschluß 12 verbunden. Das plattenförmige Gehäuseteil 24 weist in dem von dem inneren Kontaktring 29 umgebenen Bereich der Isolierplatte 27 eine Vertiefung 30 auf, der eine entsprechende Vertiefung 31 in dem schalenförmigen Gehäuseteil 25 gegenüberliegt. Beide Vertiefungen 30 und 31 bilden eine Halterung für ein aus einem Tropfen aus einer leitenden Flüssigkeit bestehendes Schaltstück 32. Die Flüssigkeit besteht aus einem die Gehäuseteile 24 und 25 nicht benetzenden Medium, wie z.B. Quecksilber, so daß sie durch ihre Oberflächenspannung zu einem Tropfen zusammengehalten wird. Auf der Oberseite des schalenförmigen Gehäuseteils 25 ist ein Piezoelement 33 aufgebracht, das zusammen mit dem Gehäuseteil 25 einen elektromechanischen Wandler bildet. Das Piezoelement 33 ist beidseitig mit den Steuereingang 22 des steuerbaren Schalters 10 bildenden Kontaktierungen versehen.

Bei der in Figur 2 gezeigten Ruhestellung des Piezoelements 33 befindet sich der Flüssigkeitstropfen 32 in einer stabilen Lage zwischen den beiden Vertiefungen 30 und 31 ohne dabei Kontakt zu dem inneren Kontaktring 29 zu haben.

Wie Figur 3 zeigt, wird bei Ansteuerung des Piezoelements 33 das schalenförmige Gehäuseteil 25 in Richtung auf das plattenförmige Gehäuseteil 24 ausgelenkt, wobei der Flüssigkeitstropfen 32 gegen die Wirkung seiner Oberflächenspannung deformiert wird. Bei einer bestimmten Auslenkung des Piezoelements 33 bzw. des schalenförmigen Gehäuseteils 25 kommt dabei der Flüssigkeitstropfen 32 in Kontakt mit dem inneren Kontaktring 29, so daß ein Stromfluß von der Seite 6 der Ladekapazität 3 über den äußeren Kontaktring 28, das schalenförmige Gehäuseteil 25, den Flüssigkeitstropfen 32 und den inneren Kontaktring 29 zu dem Elektrodenanschluß 12 möglich ist. Der dem Stromfluß dabei entgegengesetzte elektrische Widerstand ist vor allem von dem Übergangswiderstand zwischen dem Flüssigkeitstropfen 32 und dem inneren Kontaktring 29 abhängig. Je weiter das schalenförmige Gehäuseteil 25 in Richtung auf das plattenförmige Gehäuseteil 24 ausgelenkt wird, um so größer ist die Fläche, mit der der Flüssigkeitstropfen 32 mit dem inneren Kontaktring 29 in Kontakt steht. Durch zunehmende Auslenkung des schalenförmigen Gehäuseteils 25 läßt sich also der Durchgangswiderstand des steuerbaren Schalters 10 verringern.

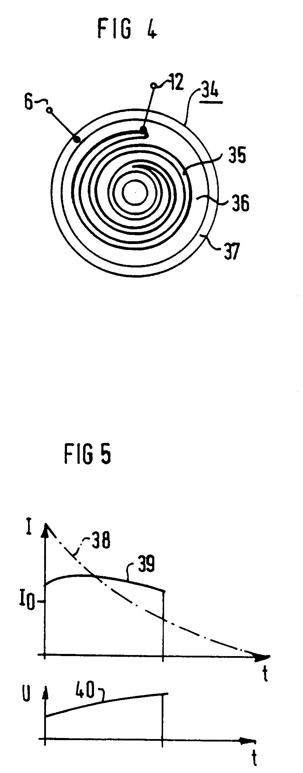

Figur 4 zeigt eine alternative Ausführung 34 des plattenförmigen Gehäuseteils 24 in Draufsicht. Dabei ist anstelle des inneren Kontaktrings 29 nach den Figuren 2 und 3 ein spiralförmiges Kontaktteil 35 in die Isolierplatte 36 eingelassen. Das spiralförmige Kontaktteil 35 ist an seiner vom Zentrum des plattenförmigen Gehäuseteils 34 am weitesten entfernten Stelle mit dem Elektrodenanschluß 12 verbunden. Die Seite 6 der Ladekapazität 3 ist dagegen wie bei dem Ausführungsbeispiel nach den Figuren 2 und 3 an einem äußeren Kontaktring 37 am äußeren Rand der Isolierplatte 36 angeschlossen. Je mehr der Flüssigkeitstropfen 32 (Figuren 2 und 3) durch das schalenförmige Gehäuseteil 25 zusammengedrückt wird, um so kürzer wird die Leitungslänge zwischen dem mit dem Elektrodenanschluß 12 verbundenen äußersten Punkt des spiralenförmigen Kontaktteils und dem Bereich des spiralförmigen Kontaktteils 35, das mit dem Flüssigkeitstropfen 32 in Verbindung steht. Auf diese Weise läßt sich mit zunehmender Auslenkung des schalenförmigen Gehäuseteils 25 der Durchgangswiderstand des Schalters 10 verringern.

Mit Hilfe der in den Figuren 2,3 und 4 gezeigen Ausführungsbeispiele für den steuerbaren Schalter 10 läßt sich bei dem Defibrillator nach Figur 1 der Entladestrom der Ladekapazität 3 begrenzen. Figur 5 verdeutlicht dies an Hand zweier Stromverläufe 38 und 39, von denen der mit 38 bezeichnete Stromverlauf eintritt, wenn die Ladekapazität 3 auf herkömmliche Weise, d.h. ohne irgendwelche Strombegrenzung, über die Elektroden 17 und 18 und das dazwischen liegende Herzgewebe 16 entladen wird. Der Stromlauf 38 weist zu Beginn der Entladung der Ladekapazität 3 seinen höchsten Wert auf und klingt danach exponentiell ab, wobei nach einer gewissen Zeit die mit I₀ bezeichnete Mindeststromstärke zur effektiven Defibrillation des Herzens 16 unterschritten wird. Wie der Figur zu entnehmen ist, kann der Anfangswert des Stromverlaufs 38 erheblich über der Defibrillationsschwelle I₀ liegen, wobei Schädigungen des Herzgewebes 16 nicht auszuschließen sind.

Demgegenüber erhält man bei Ansteuerung des steuerbaren Schalters 10 mit dem im unteren Teil von Figur 5 gezeigten Einschaltimpuls 40 den mit 39 bezeichneten Stromverlauf, bei dem der Strom am Anfang der Entladung der Ladekapazität 3 auf einen unschädlichen Maximalwert begrenzt ist und zusätzlich in vorteilhafter Weise während eines längeren Zeitraums als bei dem Stomverlauf 38 über dem zur Defibrillation erforderlichen Mindestwert I₀ liegt. Der dazu von dem Steuerimpulsgeber 23 erzeugte Einschaltimpuls 40 weist zu Beginn des Impulses eine Anfangsimpulshöhe auf, durch die der steuerbare Schalter 10 eingeschaltet wird. Dabei wird der elektrische Strom durch den Schalter 10 durch den Übergangswiderstand zwischen den Flüssigkeitstropfen 32 und den inneren Kontaktring 29 bzw. den spiralförmigen Kontaktteil 35 begrenzt. Während der Dauer des Einschaltimpulses 40 wird die Impulshöhe vergrößert, so daß dadurch der elektromechanische Wandler 25,33 noch weiter ausgelenkt wird und dadurch der elektrische Durchgangswiderstand des steuerbaren Schalters 10 verringert wird.

### Bezugszeichenliste

- 1: Spannungsquelle (Batterie)
- 2: Ladeschaltung
- 3: Ladekapazität
- 4,5: Ausgangsanschlüsse von 2
- 6,7: Seiten von 3
- 8,9: steuerbare Schalter
- 10,11: weitere steuerbare Schalter (Schalteinrichtung)
- 12,13: Elektrodenanschlüsse
- 14,15: Elektrodenleitungen
- 16: Herz
- 17,18: Elektroden
- 19,20: Steuerausgänge von 21
- 21: Steuerschaltung
- 22: Steuereingang von 10
- 23: Steuerimpulsgenerator
- 24: plattenförmiges Gehäuseteil
- 25: schalenförmigeS Gehäuseteil
- 26: Hohlraum zwischen 24 und 25
- 27: Isolierplatte
- 28: äußerer Kontaktring
- 29: innerer Kontaktring
- 30,31: Vertiefung
- 32: Schaltstück (Flüssigkeitstropfen)
- 33: Piezoelement
- 34: plattenförmiges Gehäuseteil
- 35: spiralförmiges Kontakteil
- 36: Isolierplatte
- 37: äußerer Kontaktring
- 38,39: Stromverläufe
- 40: Einschaltimpuls

## Patentansprüche

1. Defibrillator mit einer Ladekapazität (3), die an ihren beiden Seiten (6, 7) zum Aufladen an eine Ladeschaltung (2) schaltbar ist und zum Defibrillieren eines Herzens (16) über eine steuerbare Schalteinrichtung (10, 11) mit mindestens zwei im Bereich des Herzens (16) angeordneten Elektroden (17, 18) verbindbar ist, wobei die steuerbare Schalteinrichtung (10) zwei voneinander elektrisch isolierte Kontakte (28, 29, 35, 37) aufweist, **dadurch gekennzeichnet,** dass zwischen den Kontakten (28, 29, 35, 37) ein gegen die Wirkung einer inneren Rückstellkraft deformierbares und unter der Einwirkung eines elektromechanischen Wandlers (25, 33) stehendes Schaltstück (32) in der Weise angeordnet ist, dass das Schaltstück (32) bei einer vorgegebenen Auslenkung des elektromechanischen Wandlers (25, 33) die beiden Kontakte (28, 29, 35, 37) miteinander leitend verbindet und bei einer von der vorgegebenen Auslenkung des elektromechanischen Wandlers (25, 33) ausgehenden Änderung der Auslenkung des Schaltstücks (32) aufgrund der davon abhängigen Formänderung des Schaltstücks (32) den elektrischen Übergangswiderstand zwischen den Kontakten (28, 29, 35, 37), durch Änderung der in Kontakt stehenden Fläche der Kontakte (28, 29, 35, 37) verändert, und dass an dem elektromechanischen Wandler (25, 33) ein Steuerimpulsgenerator (23) angeschlossen ist, der Einschaltimpulse (40) mit einer zum Einstellen des gewünschten elektrischen Übergangswiderstands zwischen den Kontakten (28, 29, 35, 37) veränderbaren Impulshöhe erzeugt.

2. Defibrillator nach Anspruch 1, **dadurch gekennzeichnet,** dass die Impulshöhe des Einschaltimpulses (40) während der Impulsdauer in der Weise variiert, dass der elektrische Übergangswiderstand zwischen den beiden Kontakten (28, 29, 35, 37) während der Impulsdauer abnimmt.

3. Defibrillator nach Anspruch 1 oder 2, **dadurch gekennzeichnet,** dass das Schaltstück (32) bei unterschiedlichen Auslenkstellungen des elektromechahischen Wandlers (25, 33) unterschiedlich grosse Flächen oder unterschiedliche Bereiche der Kontakte (28, 29, 35, 37) bedeckt.

4. Defibrillator nach Anspruch 3**, dadurch gekennzeichnet,** dass die Kontakte (28, 29, 35, 37) aus einem Widerstandsmaterial bestehen.

5. Defibrillator nach einem der vorangehenden Ansprüche, **dadurch gekennzeichnet,** dass das Schaltstück (32) aus einer vorgegebenen Menge einer leitenden Flüssigkeit besteht.

6. Defibrillator nach einem der vorangehenden Ansprüche, **dadurch gekennzeichnet,** dass der elektromechanische Wandler (25, 33) aus einem piezoelektrischen, elektromagnetischen, magnetostriktiven oder elektrostriktiven Antriebselement besteht.

## Claims

1. Defibrillator with a loading capacitance (3) which can be connected at its two sides (6, 7) to a loading circuit (2) for charging and can be connected by way of a controllable switching device (10, 11) to at least two electrodes (17, 18), arranged in the region of a heart (16), for the defibrillation of the heart (16), with the controllable switching device (10) having two contacts (28, 29, 35, 37) electrically insulated from each other, characterized in that between the contacts (28, 29, 35, 37) a contact piece (32), which can be deformed against the action of an inner restoring force and which is under the effect of an electromechanical transformer (25, 33), is arranged in such a way that upon a specified displacement of the electromechanical transformer (25, 33) the contact piece (32) conductively connects the two contacts (28, 29, 35, 37) to each other and, upon a change of the displacement of the contact piece (32), which change proceeds from the specified displacement of the electromechanical transformer (25, 33), because of the deformation of the contact piece (32) depending thereon, changes the electrical transition resistance between the contacts (28, 29, 35, 37) by changing the surface of the contacts (28, 29, 35, 37) which is in contact, and in that a control pulse generator (23) is connected to the electromechanical transformer (25, 33), which control pulse generator generates connecting pulses (40) with a pulse height which can be varied for the setting of the desired electrical transition resistance between the contacts (28, 29, 35, 37).

2. Defibrillator according to claim 1, characterized in that the pulse height of the connecting pulse (40) for the duration of the pulse varies in such a way that the electrical transition resistance between the two contacts (28, 29, 35, 37) decreases for the duration of the pulse.

3. Defibrillator according to claim 1 or 2, characterized in that with different displacement positions of the electromechanical transformer (25, 33) the contact piece (32) covers surfaces of different sizes or different areas of the contacts (28, 29, 35, 37).

4. Defibrillator according to claim 3, characterized in that the contacts (28, 29, 35, 37) consist of a resistance material.

5. Defibrillator according to one of the preceding claims, characterized in that the contact piece (32) consists of a specified quantity of a conducting fluid.

6. Defibrillator according to one of the preceding claims, characterized in that the electromechanical transformer (25, 33) consists of a piezoelectric, electromagnetic, magnetostrictive or electrostrictive driving element.

## Revendications

1. Défibrillateur comportant une capacité (3) de charge qui, pour être chargée, peut être connectée de ses deux côtés (6, 7) à un circuit (2) de charge et qui, pour la défibrillation d'un coeur (16), peut, par l'intermédiaire d'un dispositif (10, 11) de coupure pouvant être commandé, être reliée à au moins deux électrodes (17, 18) montées dans la région du coeur (16), le dispositif (10) de coupure pouvant être commandé comportant deux contacts (28, 29, 35, 37) isolés l'un de l'autre du point de vue électrique, caractérisé en ce qu'il est monté entre les contacts (28, 29, 35, 37) une pièce (32) de coupure pouvant être déformée à l'encontre d'une force de rappel intérieure et soumise à l'influence d'un transformateur (25, 33) électromécanique, de telle manière que, pour une déviation prescrite du transformateur (25, 33) électromécanique, la pièce (32) de coupure relie entre eux les deux contacts (28, 29, 35, 37) de manière conductrice et que, pour une modification de la déviation de la pièce (32) de coupure à partir de la déviation prescrite du transformateur (25, 33) électromécanique en raison du changement de forme qui en dépend de la pièce (32) de coupure, la pièce (32) de coupure modifie la résistance électrique de contact entre les contacts (28, 29, 35, 37) en modifiant la surface de contact des contacts (28, 29, 35, 37), et en ce qu'il est raccordé au transformateur (25, 33) électromécanique un générateur (23) d'impulsion de commande qui produit des impulsions (40) de fermeture ayant un niveau d'impulsion pouvant être modifié pour régler la résistance électrique de contact souhaitée entre les contacts (28, 29, 35, 37).

2. Défibrillateur suivant la revendication 1, caractérisé en ce que le niveau d'impulsion de l'impulsion (40) de fermeture varie pendant la durée de l'impulsion, de telle manière que la résistance électrique de contact entre les deux contacts (28, 29, 35, 37) diminue pendant la durée de l'impulsion.

3. Défibrillateur suivant la revendication 1 ou 2, caractérisé en ce que la pièce (32) de coupure couvre des surfaces de dimensions différentes ou des zones différentes des contacts (28, 29, 35, 37) pour des positions de déviation différentes du transformateur (25, 33) électromécanique.

4. Défibrillateur suivant la revendication 3, caractérisé en ce que les contacts (28, 29, 35, 37) sont en un matériau résistif.

5. Défibrillateur suivant l'une des revendications précédentes, caractérisé en ce que la pièce (32) de coupure est constituée d'une quantité prescrite d'un liquide conducteur.

6. Défibrillateur suivant l'une des revendications précédentes, caractérisé en ce que le transformateur (25, 33) électromécanique est constitué d'un élément d'entraînement piézo-électrique, électromagnétique, magnétostrictif ou électrostrictif.
